# EUROPEAN PATENT APPLICATION

(11) **EP 4 296 677 A1**
(43) Date of publication of application: **27.12.2023**
(21) Application number: 22180522.9
(22) Date of filing: 22.06.2022
(51) Int. Cl.: G01N 33/68, G16H 10/00

(54) **METHOD FOR DETERMINING THE THERAPEUTIC REGIMEN FOR AN INDIVIDUAL AND ITS USE**

(71) Applicant: Mosaiques Diagnostics And Therapeutics AG, 30659 Hannover (DE)
(72) Inventor: Mischak, Harald, Hannover (DE)
(74) Representative: Kröncke, Rolf

(57) **Abstract**

The present invention relates in a first aspect to a method for determining the therapy regimen and/or the clinical outcome and/or the treatment success of a treatment of an individual either prophylactically or therapeutically with an active agent, whereby the peptidome/proteome in a body fluid sample from the individual is determined and a reference value is calculated based on processing the information of a predetermined group of peptide/protein markers present in the peptidome/proteome determined and determining a score for the predetermined group of peptide/protein markers, whereby the score is calculated by processing information for each marker of the group obtained under the impact of an active agent whereby the information is present in a database and processing the value of the individual with a score, thus, determining a therapy regimen and/or predicting the clinical outcome and/or predicting the treatment success with the active agent based on the processing. That is, the method allows to predict whether an active agent is beneficial for the treatment of the individual to be analysed. The method is particularly useful in predicting very relevant health event and determining possible preventive treatment to counteract the possible very relevant health event in the future. In addition, a computer implemented method is provided as well as a computer readable medium or computer program product and the use of a test kit with the method according to the present invention.

## Description

The present invention relates in a first aspect to a method for determining the therapy regimen and/or the clinical outcome and/or the treatment success of a treatment of an individual either prophylactically or therapeutically with an active agent, whereby the peptidome/proteome in a body fluid sample from the individual is determined and a reference value is calculated based on processing the information of a predetermined group of peptide/protein markers present in the peptidome/proteome determined and determining a score for the predetermined group of peptide/protein markers, whereby the score is calculated by processing information for each marker of the group obtained under the impact of an active agent whereby the information is present in a database and processing the value of the individual with a score, thus, determining a therapy regimen and/or predicting the clinical outcome and/or predicting the treatment success with the active agent based on the processing. That is, the method allows to predict whether an active agent is beneficial for the treatment of the individual to be analysed. The method is particularly useful in predicting a very relevant health event and determining possible preventive treatment to counteract the possible very relevant health event in the future. In addition, a computer implemented method is provided as well as a computer readable medium or computer program product and the use of a test kit with the method according to the present invention.

### Prior art

Urine contains more than 20,000 endogenous peptides or proteins which are partly generated along the nephron. The value of the urinary peptidomic profile has been demonstrated in various publications and for various approaches. For example, the urinary peptidomic profile predicts outcome in SARS-CoV-2 infected patients, see Wendt R., et al., E Clinical Medicine 2021;36:100883. That is sequencing these urinary peptides identifies parental proteins and the urinary peptidomic profile (UPP) provides a body wide molecular signature of ongoing pathophysiological processes. For example, UPP generated molecular signatures have been shown to be associated with a preclinical phase of heart failure, chronic kidney disease, diabetic nephropathy and a variety of other disorders, see e. g Zhang et al Hypertension. 2015 Jul;66(1):52-60 or Zhang et al. Proteomics Clin Appl.. 2019 Mar;13(2):e1800174.. For example, Martens et al., Lancet Healthy Longev. 2021 Nov;2(11):e690-e703 refers to urinary peptidomic profiles to address age related disabilities and provides a prospective population study to identify and validate a UPP signature that differentiates healthy from unhealthy agents in a population cohort with long term follow up; to replicate the trained UPP agent clocking patients and validate the clock in the general population as a correlate or predictor of adverse health outcomes; and to detect the molecular pathways implicated in unhealthy agents.

In particular, Martens et al. identify that the UPP signature described therein is indicative of aging reflects fibrosis and extracellular matrix modelling and was associated with risk factors and adverse health outcomes in the population and with accelerated aging in patients. Further, He. T. et al., Clin. Transl. Med., 2021, 11, e267, identifies serum and urinary biomarkers of collagen type I turnover predicting prognosis in patients with heart failure.

However, there is still an ongoing need for methods for enabling predictions of the survival rate of individuals within a predetermined time period or at a predetermined time end point, in particular, in case of healthy individuals prospective predictability of adverse events, in particular, death, is desired to allow preventive measures including pharmaceutical and physical interference.

Moreover, although UPP signatures to predict various outcomes in a predefined correlation, the prediction is not sufficient to identify suitable therapeutic regimen nor allow to predict treatment success based on treatment therapies. That is, although a large number of specific endogenous peptides can be detected in urine, an association with onset and progression of specific chronic diseases, like chronic kidney disease, coronary artery disease, heart failure or malignant tumors and also death may be allowed, the treatment success either prophylactically or therapeutically, cannot be predicted.

Thus, an object of the present invention is to provide a method allowing prediction of therapy success in treating an individual afflicted with a disease, disorder or condition or of individuals although not diagnosed for a specific disease, but may develop a disease based on the UPP determined.

In particular, an object of the present invention is to provide a method allowing personalised treatment of individuals in need thereof.

### Brief description of the present invention

In a first aspect, the present invention relates to a method determining the therapy regimen and/or the clinical outcome and/or the treatment success of a treatment of an individual with an active agent, comprising the steps of
a) Providing a body fluid sample from the individual;
b) Determining the peptidome/proteome in said sample from said individual;
c) Calculating of a reference value based on processing information of a predetermined group of peptide/protein markers of the peptidome/proteome determined in step b);
d) Determining a score for the predetermined group of peptide/protein markers of step c), whereby the score is calculated by processing information for each marker of the group obtained under the impact of the active agent, whereby the information is present in a database;
e) Processing the value obtained in step c) with the score obtained in step d);
f) Determining a treatment regimen and/or predicting the clinical outcome and/or predicting the treatment success with the active agent based on the processing in step e), whereby if the score is above the reference value, the treatment success is predicted to be positive and/or the therapeutic regimen shall be applied and/or the clinical outcome is favourable.

In a further aspect, a method for the prediction of a very relevant health event for an individual and the determination of possible preventive treatment of said individual, comprising firstly, the steps for the probability of a very relevant health event by
a) Providing a body fluid sample from the individual;
b) Determining the peptidome/proteome in said sample;
c) Comparing the information on the peptidome/proteome obtained in step b) with information on the peptidome/proteome present in a database;
d) Determining the probability of a very relevant health event for an individual based on the comparison in step c); and, secondly,
e) Calculating of a reference value based on processing information of a predetermined group of peptide/protein markers determined in step b);
f) Determining a score for the predetermined group of peptide/protein markers used in step b), whereby the score is calculated by processing information for each marker of the group obtained under the impact of the active agent, whereby the information is present in a database;
g) Processing the value obtained in step e) with the score obtained in step f);
h) Determining a treatment regimen and/or predicting the clinical outcome and/or predicting the treatment success with the active agent based on the processing in step g), whereby if the score is above the reference value, the treatment success is predicted to be positive and/or the therapeutic regimen shall be applied and/or the clinical outcome is favourable,
is provided.

Moreover, the present invention relates to the use of a test kit, or kit of parts for the prediction of a very relevant health event for an individual; for determining the therapeutic regimen; for predicting the clinical outcome; and/or for predicting the treatment success of a treatment for an individual, respectively, said kit comprising means for determining the peptidome/proteome present in a body fluid sample and, optionally, means for reprocessing of the body fluid sample before determining the peptidome/proteome and instructions on how to use said test kit or kit of parts for a method according to the present invention.

Finally, a computer implemented method of a very relevant health event for an individual; for determining the therapeutic regimen; for predicting the clinical outcome; and/or for predicting the treatment success of a treatment for an individual with an active agent, respectively, comprising the steps of
a) Obtaining information of the peptidome/proteome in a body fluid sample of the individual;
b) Computing the information of step a) by
   i. Comparing the information on the peptidome/proteome obtained in step a) with information on the peptidome/proteome obtained from a database; or
   ii. by calculating of a reference value based on the information of a predetermined group of peptide/protein markers and computing a score for the predetermined group of peptide/protein markers, whereby the score is calculated from information for each marker of the group under the influence of the active agent, whereby the information is obtained from a database, comparing the computed reference value with the score,
c) Determining the probability of a very relevant health event of an individual; or determining a treatment regimen and/or predicting the clinical outcome and/or predicting the treatment success with the active agent, respectively;
d) Optionally further comprising the step of showing the data of prediction of a very relevant health event for an individual, treatment regimen, predicting the clinical outcome and/or predicting the treatment success with the active agent, respectively, on an output unit,

Is provided as well as a computer readable medium or computer program product having computer executable instructions for performing the method according to the present invention.

### Brief description of the drawings

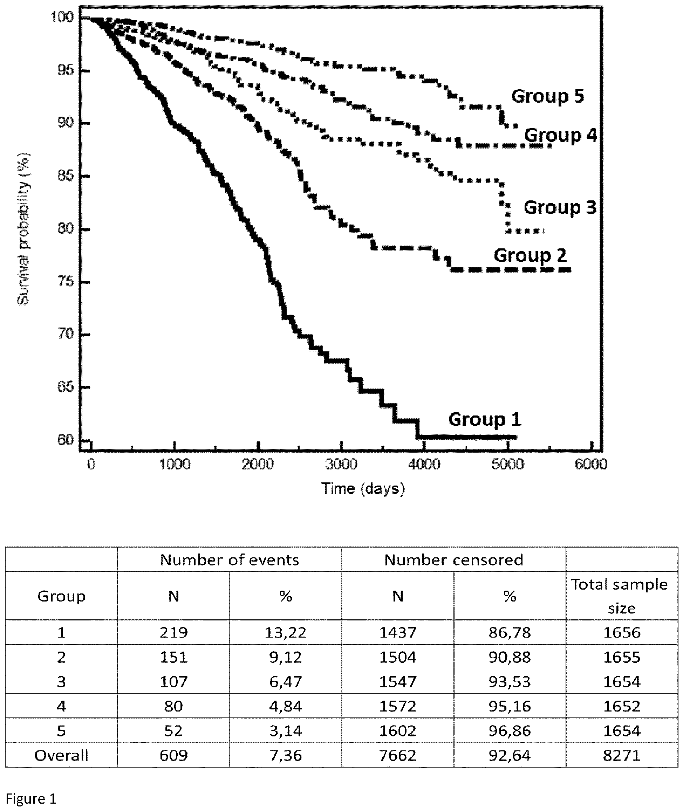

Figure 1 is the survival probability over time of a cohort of 8271 participants, whereby the participants are separated into five groups according to the classifier scoring, showing the survival probability for each of the classified groups accordingly. The
table of figure 1 shows the classification of the different groups, showing the possible classification based on the classifier into the groups discriminating the survival probability accordingly.

### Detailed description of the present invention

In a first aspect the present invention relates to a method determining the therapy regimen and/or the clinical outcome and/or the treatment success of a treatment of an individual with an active agent, comprising the steps of
a) Providing a body fluid sample from the individual;
b) Determining the peptidome/proteome in said sample from said individual;
c) Calculating of a reference value based on processing information of a predetermined group of peptide/protein markers of the peptidome/proteome determined in step b);
d) Determining a score for the predetermined group of peptide/protein markers of step c), whereby the score is calculated by processing information for each marker of the group obtained under the impact of the active agent, whereby the information is present in a database;
e) Processing the value obtained in step c) with the score obtained in step d);
f) Determining a treatment regimen and/or predicting the clinical outcome and/or predicting the treatment success with the active agent based on the processing in step e), whereby if the score is above the reference value, the treatment success is predicted to be positive and/or the therapeutic regimen shall be applied and/or the clinical outcome is favourable.

In this connection, the term "reference value" refers to a numerical value, e.g. expressing the classifier, see below.

The term "score" as used herein refers to the result of applying the predefined high-dimensional classifier to generate a dimensionless score based on preselected urine peptides/proteins

The term "therapy regimen" as used herein refers to a predefined therapeutic intervention, which could e.g. be a specific drug, specific exercise, specific lifestyle intervention, specific diet, etc.

The term "clinical outcome" refers to the result of therapy applied.

The term "treatment success of a treatment of an individual" refers to an alleviation of the disease, disorder or condition or reducing the probability of future very relevant health events.

The term "very relevant health event" as used herein refers to a relevant endpoint like death, kidney failure, major cardiovascular event, tumor recurrence, etc occurring typically in the future.

Further, the term "peptidome/proteome" refers to all detectable proteins and peptides in a sample with a molecular mass between 800 and 20000 Da.

In addition, the term "survival rate" expresses the probability, typically in percent or as a score, being alive at the predetermined time end point or staying alive within the predetermined time period. In other words, the survival rate also identifies a possible future death in percentage accordingly. As said, the survival rate is typically identified in a percentage of survival rate or in reversed expression as percentage of decease.

As used herein, the term "classifier" refers to a numeric variable that is based on a multitude of predefined peptides or proteins (peptide/protein markers) determinable in a urinary sample. The classifier has been obtained typically by applying suitable algorithms, like artificial intelligence, e. g. using machine learning algorithms.

The term "under the impact of the active agent" refers data and information obtained from individuals which have received the respective active agent. The present inventors recognized that the peptidome/proteome in a body fluid sample, in particular, in a urine sample of an individual does not only allow to predict an outcome of a known or unknown disease the individual is afflicted with but allows further to identify suitable treatment courses and effective treatment regimen as well as predicting the treatment success of a treatment of an individual with an active agent.

Typically it is possible to treat specific disease with different classes of active ingredients and selection and therapy plan of the active agent is not only based on the experience of the treating clinical expert (e.g. a physician). However, it is well known that the treatment success of a disease depends on the accessibility to the treatment selected. Today only some time (weeks, months, even years) after start of a therapy a treatment response can be identified and it is well known that not all individuals respond in the same way to a treatment with an active agent.

The inventors surprisingly recognized that the method according to the present invention allows to determine in advance the responsiveness of the individual to an active agent, based on a predetermined peptide/protein markers also referred to as classifier. Namely, when obtaining a reference value of said classifier based on the peptidome/proteome analysis of a sample from the individual with a score for the same peptide/protein markers of the classifier from a database, whereby these information or data for each of the markers is obtained from individuals treated successfully for the active ingredient, it is possible to predict a therapy success or the clinical outcome of treatment with the very same active agent. That is, the method according to the present invention allows to identify the most promising therapy regimen or therapy plan based on an active agent or principle in advance, typically, addressing a specific mechanism in the metabolism of the individual to treat therapeutically or prophylactically the individual.

Not to be bound to theory when calculating the reference value of the individual and calculating the score of the same marker or the respective classifier from the database under the impact of the active agent, the processing of these two values allows selecting a specific active agent. Namely, if the score is above the reference value, it is likely that the treatment success is positive and that the active ingredient is beneficial for treating the individual. Rather, in case when modelling the impact of the active agent, a score below or similar to the reference value, it is likely that a negative impact or no treatment success can be achieved. In particular, a negative impact which means worsening the disease or the outcome of a very relevant health event should be avoided. In other words, the method according to the present invention promotes personalised medicine insofar that the type of treatment is determined *in silico* in advance.

In an embodiment of the present invention, the method is a method for the prediction of a very relevant health event for an individual and the determination of possible preventive treatment of said individual, comprising firstly, the steps for the probability of a very relevant health event by
a) Providing a body fluid sample from the individual;
b) Determining the peptidome/proteome in said sample;
c) Comparing the information on the peptidome/proteome obtained in step b) with information on the peptidome/proteome present in a database;
d) Determining the probability of a very relevant health event for an individual based on the comparison in step c); and, secondly,
e) Calculating of a reference value based on processing information of a predetermined group of peptide/protein markers determined in step b);
f) Determining a score for the predetermined group of peptide/protein markers of step c), whereby the score is calculated by processing information for each marker of the group obtained under the impact of the active agent, whereby the information is present in a database;
g) Processing the value obtained in step e) with the score obtained in step f);
h) Determining a treatment regimen and/or predicting the clinical outcome and/or predicting the treatment success with the active agent based on the processing in step g), whereby if the score is above the reference value, the treatment success is predicted to be positive and/or the therapeutic regimen shall be applied and/or the clinical outcome is favourable.

That is, based on the prediction of a very relevant health event, it is possible to postulate a preventive and therapeutical treatment of said individual to reduce the risk of having the very relevant health event in the future.

In an aspect of the present invention, the method according to the present invention contains additionally a reprocessing of the sample before determining the peptidome/proteome in said sample from said individual.

In an embodiment, the step of comparison as identified in step c) above includes the application of a classifier, comprising at least three, like at least five, at least ten, for example at least twenty, at least thirty or more predetermined peptide/protein markers. As shown in the example below, the classifier contains more than seventy peptides, here seventy one peptides, allowing future prognosis of a very relevant health event including survival rate and death.

Namely, in one aspect, the very relevant health event may be the occurrence of death. However, according to the method of the present invention, it is possible to predict the survival rate or, in other words, a future risk of death based on the information on the peptidome/proteome of the individual, whereby the information, in a preferred embodiment, a scoring of a specific predetermined multidimensional classifier, is compared with information of the same peptidome/proteome present in a database. Based on the comparison of the scoring of the classifier, a survival rate of said individual for predetermined time periods or at the predetermined time end point can be predicted. This method for predicting the survival rate or a risk of death is particularly helpful in establishing preventive treatment, either by physical means or by pharmaceutical treatment.

Based on this prediction of survival rate, the suitability of active agents for decreasing the possibility of the event of death, thus, increasing the survival rate can be determined by the present invention.

The polypeptide markers or protein markers according to the present invention are proteins or peptides, including specific and defined (by their amino acid sequence) degradation products of proteins or peptides. These markers may be chemically modified, for example by posttranslational modifications, such as glycosylation, phosphorylation, hydroxylation, oxidation, alkylation, carbamylation or disulfide bridges or by other reactions, for example, within the scope of the degradation. Apart from the parameters that determine the polypeptide markers, e. g. the molecular weight and migration time when applying a CE-MS method, it is possible to identify the sequence of the corresponding polypeptides by method known in the art, typically using tandem mass spectrometry, e.g. as described above by Wemdft et. al.

As said, the method is based on the information obtained from the body fluid peptidome/proteome in the sample of the individual.

The present inventors recognized that applying the method of the present invention allows to determine a risk of death of the individual, although said individual may be apparently healthy or may suffer from a non-life threatening disease. The adverse event behind the risk of death may not yet be determined in said individual or may be connected with a pre-existing illness. On the other hand, the adverse event for a risk of death may be independent from a pre-existing illness. The predefined multidimensional classifier according to the present invention based on the body fluid, like the urinary peptidome/proteome allows to determine the survival rate or the risk of death of the individual when compared with data from a database. The data from the database may allow to classify individuals into individual groups of having a different risk of death.

In particular, the scoring of the classifier by comparing the information with information from a database may be by applying suitable algorithms, like an artificial intelligence routine. In this connection, the artificial intelligence routine may include a neural network (in general), like convolutional neural networks. For example, the neural network can be provided with trained weight-in factors or can be trained at use. For example, the multidimensional classifier is obtained with machine learning algorithms That is, suitable methods and algorithms include SVM, uMAP, or decision trees. The peptide/protein marker of the peptidome/proteome present in the sample of the individual are combined to the classifier as described herein, whereby the peptides/proteins are associated with a future death.

In an embodiment of the present invention, the steps of comparing the information and determining the survival rates according to step c) and d) described above may be conducted with artificial intelligence routine. As said above, the artificial intelligence routine may include a neural network. In an embodiment, the comparison of the information, in particular, of the classifier may be conducted by machine learning algorithm, in particular, by support vector machine.

The classifier according to the present invention is developed on the basis of data obtained from cohorts of subjects, including information on survival rate or risk of death based on the peptidome/proteome present in the sample.

In an embodiment, the sample is a urine sample and the peptidome/proteome is present in the urinary sample accordingly. For example, the urine sample is a midstream urine sample from the individual.

The classifier applied according to the present invention is developed typically from data of cohorts of subjects or individuals with known clinical history. The proteins/peptides included in the classifiers are significantly associated with future death or with a survival rate of the individual accordingly. As said, the multidimensional classifier may be developed using machine learning algorithms and may be further refined by optimising with a take one out procedure known to the skilled person, as described in Wendt R., et al., E Clinical Medicine 2021;36:100883.

In an embodiment, the method according to the present invention is applicable for individual wherein the individuals are apparently healthy individuals, or the individuals are afflicted with a known disease.

In an embodiment, this predefined classifier is applied onto the individual or a number of individuals for classifying these numbers of individuals into different groups or for predicting survival rate and risk of death of the individual and the cohort accordingly. Namely, a highly significant predictive power is demonstrated with the classifier enabling establishment of correlation between the risk of future death and the score obtained.

In this connection, the score is expressed as dimensionless numeric variable with known and defined association to the survival rate.

In an embodiment of the present invention, the method for the prediction of the survival rate of the individual includes the comparison by evaluating of a determined presence or absence or amplitude of the peptide/protein markers, for example, the classifier predefined composed of a multitude of predefined peptide/protein markers.

In an embodiment, the information of the peptidome/proteome, in particular, the predetermined group of peptide/protein markers include values for the molecular masses, migration time, in particular obtained by CE-MS, and/or the information includes an adjusted p-value for the association with future death or survival rate at predetermined time end points or within a predetermined time periods.

In an embodiment, the method according to the present invention comprises applying algorithms, like an artificial intelligence routine. Namely, the artificial intelligence routine is applied with information of the sequence of the peptidome/proteome, determined, in particular, of the predetermined peptide/protein markers as input data and information on the survival rate of said individual or the risk of future death as output information.

In an embodiment, the determination of the peptidome/proteome of the body fluid sample, in particular, the urinary sample is obtained by capillary electrophoresis, HPLC, gas-phase, ion spectrometry, in combination with mass spectrometry. The methods are well known to the skilled person accordingly.

In an embodiment of the present invention, the peptide/protein markers determined in the body fluid sample, are urinary peptide/protein markers including collagen-derived peptides/proteins, in particular, collagen α-1-derived peptides/proteins.

Further, in an embodiment of the present invention the method for prediction of the survival rate of an individual according to the present invention includes a prediction of an occurrence of possible diseases within the predetermined time period or at a predetermined time end point. That is, the occurrence of a disease may include an adverse health event. This adverse event may be an acute adverse event or may be a chronic adverse event. In addition, this adverse event may be a life threatening adverse event, in particular, may be an adverse event eventually resulting in death of the individual. The occurrence of the possible adverse event may be due to a pre-existing illness of the individual or may be due to a not yet determined illness of the individual or may be due to a new adverse event.

For example, the method according to the present invention may be applied for individuals under particular situations. For example, it may be of interest to receive information on the survival rate on persons or individuals within intensive care units (ICU) or with familiar predisposition of a specific disease or with genetic predisposition.

In an embodiment, the method according to the present invention for the prediction of the survival rate of an individual is a method wherein the individual may be a healthy individual or may be an individual afflicted with a known disease and the survival rate is reduced due to a different, not yet diagnosed disease.

In the method according to the present invention, the determination of the peptidome/proteome including a characterisation of the peptide/protein markers may be determined by means of capillary electrophoresis-mass spectrometry, a method which has been described in detail previously, see e. g. E Mavrogeorgis et al., Molecules, 2021;26(23):7260

Moreover, the database used according to the present invention contains data of a multitude of peptidome/proteome information of individuals either in form of the raw data or processed data, e. g. identifying specific peptides/proteins in combination with further information. For example, this information includes information on association with known adverse events including death.

In an embodiment, the method refers to a very relevant health event whereby this very relevant health event is selected from major cardiovascular events, kidney failure, tumor recurrence or death as a result of a disease. Moreover, the method according to the present invention is a method predicting the treatment success with a predetermined active agent *in silico* for stratification of the therapeutic regimen. Stratification of the therapeutic regimen means that the therapy regimen as well as the treatment success or the clinical outcome is analysed before applying or starting the therapy as well as during therapy.

It is submitted that for determining the therapy with an active agent, the score of the classifier based on the peptide/protein markers under impact of the active agent is at or below the reference value of the classifier, the administration of the active agent does not have any additional therapeutic effect.

In an embodiment of the present invention, the method comprises application of an artificial intelligence network, wherein the artificial intelligence network is supplied with information of the sequence of the peptidome/proteome determing, in particular, the predetermined peptide/protein markers as input data and information and at least one of the prediction of a very relevant health event for an individual; for determining the therapeutic regimen; for predicting the clinical outcome; and/or for predicting the treatment success of a treatment for an individual with an active agent, respectively, as output information.

In another aspect, the present invention relates to the use of a test kit or kit of parts for the prediction of a very relevant health event for an individual; for determining the therapeutic regimen; for predicting the clinical outcome; and for predicting the treatment success of a treatment of an individual, respectively, in particular for evaluating the treatment with a predetermined active agent, said kit comprising means for determining the peptidome/proteome present in a body fluid sample, like urine, and, optionally, means for reprocessing of the body fluid sample before determining the peptidome/proteome as well as instructions on how to use said test kit or kit of parts in a method according to the present invention. Moreover, the present invention relates to a computer implemented method of prediction of a very relevant health event for an individual; for determining the therapeutic regimen; for predicting the clinical outcome; and/or for predicting the treatment success of a treatment for an individual with an active agent, respectively, comprising the steps of
a) Obtaining information of the peptidome/proteome in a body fluid sample of the individual;
b) Computing the information of step a) by
   i. Comparing the information on the peptidome/proteome obtained in step a) with information on the peptidome/proteome obtained from a database; or
   ii. by calculating of a reference value based on the information of a predetermined group of peptide/protein markers and computing a score for the predetermined group of peptide/protein markers, whereby the score is calculated from information for each marker of the group under the influence of the active agent, whereby the information is obtained from a database, comparing the computed reference value with the score,
c) Determining the probability of a very relevant health event of an individual; or determining a treatment regimen and/or predicting the clinical outcome and/or predicting the treatment success with the active agent, respectively;
d) Optionally further comprising the step of showing the data of prediction of a very relevant health event for an individual, treatment regimen, predicting the clinical outcome and/or predicting the treatment success with the active agent, respectively, on an output unit.

The computing according to step b) may include the application of suitable algorithms, like artificial intelligence routine as described for the method according to the present invention above. For example, the computing includes the generation of a scoring based on a predefined multidimensional classifier and comparing the score of said classifier with scores obtained from cohorts of individuals analysed and classified before. Analysis and classification is conducted particularly with respect to adverse events including death. Thus, the score obtained allows to determine the survival rate or the risk of death within a predetermined time period.

The computing may include suitable algorithms, like SVM, uMAP, decision trees, but also neural network with training algorithms, if required.

Finally, the present invention provides a computer readable medium or computer program product having computer executable instructions for performing the steps according to the present invention.

### Example 1:

Analysis of a cohort of a multitude of subjects from various studies, identifying the survival rate of said individuals and, in addition, applying the method according to the present invention based on the CD71 classifier described, analysing the impact of specific drugs

A urine sample is prepared according to standard protocol and its proteome/peptidome contents are analysed as described, see below. This results in a list of 3920 peptides and proteins with defined abundance in the sample.

In previous studies in cohorts of over 1000 subjects, a classifier predicting the risk of death was developed. In this previous study, proteins/peptides significantly associated with future death were identified. These were combined into a high-dimensional classifier, using machine learning algorithms. The classifier was further refined by optimizing with a take-one-out procedure to contain 71 peptides (termed CD71):
Applying this classifier onto a cohort of 8271 subjects demonstrated a highly significant predictive power and enabled establishing a correlation between the risk of future death and the classification score.

To assess the risk of death of the subject giving the sample, the abundance information on the 71 predefined peptides is investigated:

| SEQ.IDNo. Peptide/protein ID | Amplitude |
|---|---|
| 1. 99900220 | 376,93 |
| 2. 99900467 | 459,22 |
| 3. 99901399 | 1375,51 |
| 4. 99902592 | 795,29 |
| 5. 99903025 | 5900,22 |
| 6. 99904405 | 0 |
| 7. 99904666 | 0 |
| 8. 99904862 | 440,81 |
| 9. 99905044 | 192,86 |
| 10.99905259 | 2467,51 |
| 11.99905274 | 2023,58 |
| 12.99905379 | 936,39 |
| 13. 99905423 | 1284,7 |
| 14.99905509 | 0 |
| 15.99905551 | 5424,19 |
| 16.99906176 | 338,51 |
| 17.99907275 | 813,21 |
| 18.99907807 | 1255,48 |
| 19.99908755 | 1289,66 |
| 20.99908795 | 0 |
| 21.99908916 | 1078,22 |
| 22.99909059 | 601,7 |
| 23.99910002 | 0 |
| 24.99910032 | 2530,19 |
| 25.99910407 | 0 |
| 26.99910483 | 1897,03 |
| 27.99910554 | 8095,41 |
| 28.99910883 | 231,38 |
| 29.99911002 | 1913,47 |
| 30.99911193 | 1263,31 |
| 31.99911415 | 316,09 |
| 32.99911559 | 1267,45 |
| 33.99911777 | 146,72 |
| 34.99912101 | 656,51 |
| 35.99912211 | 588,78 |
| 36.99912366 | 454,24 |
| 37.99912411 | 343,73 |
| 38.99912661 | 4149,61 |
| 39.99913200 | 375,62 |
| 40.99913322 | 0 |
| 41.99913825 | 1135,47 |
| 42.99914211 | 635,96 |
| 43.99914445 | 163,97 |
| 44.99914876 | 641,34 |
| 45.99914887 | 0 |
| 46.99914893 | 0 |
| 47.99914935 | 58,85 |
| 48.99915015 | 0 |
| 49.99915755 | 0 |
| 50.99915778 | 105,04 |
| 51.99915883 | 147,94 |
| 52.99916170 | 2954,99 |
| 53.99916177 | 10106,78 |
| 54.99916611 | 0 |
| 55.99916615 | 0 |
| 56.99916966 | 1033,15 |
| 57.99917047 | 6484,27 |
| 58.99917221 | 49,54 |
| 59.99917280 | 943,09 |
| 60.99917288 | 1468,46 |
| 61.99917419 | 416,27 |
| 62.99917505 | 261,69 |
| 63.99917596 | 560,74 |
| 64.99917626 | 656,75 |
| 65.99917690 | 2383,49 |
| 66.99917825 | 257,55 |
| 67.99917890 | 825,93 |
| 68.99917947 | 567,78 |
| 69.99918831 | 201,86 |
| 70.99918906 | 295,19 |
| 71.99918909 | 127,29 |

Applying the CD71 classifier based on these 71 biomarkers results in a score of 1.601, which reflects a chance of death within the next 5 years of 3.44%. This is a significantly increased risk, in comparison to the general population at the same age (male, 51 years old). Adding additional relevant variables, age, blood pressure eGFR, LDL, HDL, blood glucose and number of comorbidities results in an adjusted risk of 3.41 %, still higher than the expected average.

In the next step, the impact of specific drugs is modelled and predicted. Based on the data available in the database from previous studies where the impact of drugs was investigated, the impact of specific drugs, A, B, and C in this example, on the abundance of all known and detected urinary proteins/peptides, including these 71 biomarkers, is known, and can be expressed as fold-change of the abundance. Multiplying the actual value for a specific peptide with the predicted fold-change for a drug will generate a new, predicted value, as shown in the example:

| Seq.ID.No./Peptide /protein ID | Amplitude | f Cand. | pred drug A | f Spiro | pred drug B | f Cana | pred drug C |
|---|---|---|---|---|---|---|---|
| 1. 99900220 | 376,93 | 1 | 376,93 | 1 | 376,93 | 1 | 376,93 |
| 2. 99900467 | 459,22 | 1 | 459,22 | 1 | 459,22 | 1 | 459,22 |
| 3. 99901399 | 1375,51 | 1 | 1375,51 | 1 | 1375,51 | 1 | 1375,51 |
| 4. 99902592 | 795,29 | 1 | 795,29 | 1 | 795,29 | 1 | 795,29 |
| 5. 99903025 | 5900,22 | 1 | 5900,22 | 1 | 5900,22 | 1 | 5900,22 |
| 6. 99904405 | 0 | 1 | 0 | 1 | 0 | 1,22 | 0 |
| 7. 99904666 | 0 | 1 | 0 | 1 | 0 | 1 | 0 |
| 8. 99904862 | 440,81 | 1,4 | 617,134 | 1 | 440,81 | 1 | 440,81 |
| 9. 99905044 | 192,86 | 1 | 192,86 | 1 | 192,86 | 1 | 192,86 |
| 10.99905259 | 2467,51 | 1,5 | 3701,265 | 1 | 2467,51 | 1 | 2467,51 |
| 11.99905274 | 2023,58 | 1 | 2023,58 | 1 | 2023,58 | 1,86 | 3763,8588 |
| 12.99905379 | 936,39 | 1 | 936,39 | 1 | 936,39 | 1 | 936,39 |
| 13.99905423 | 1284,7 | 1,15 | 1477,405 | 1 | 1284,7 | 1 | 1284,7 |
| 14.99905509 | 0 | 1 | 0 | 0,77 | 0 | 1 | 0 |
| 15.99905551 | 5424,19 | 1 | 5424,19 | 1,97 | 10685,6543 | 1 | 5424,19 |
| 16.99906176 | 338,51 | 1,47 | 497,6097 | 1 | 338,51 | 1 | 338,51 |
| 17.99907275 | 813,21 | 1 | 813,21 | 1 | 813,21 | 1 | 813,21 |
| 18.99907807 | 1255,48 | 1 | 1255,48 | 1 | 1255,48 | 1,84 | 2310,0832 |
| 19.99908755 | 1289,66 | 1 | 1289,66 | 1 | 1289,66 | 1 | 1289,66 |
| 20.99908795 | 0 | 1,49 | 0 | 1 | 0 | 1 | 0 |
| 21.99908916 | 1078,22 | 1 | 1078,22 | 1 | 1078,22 | 1 | 1078,22 |
| 22.99909059 | 601,7 | 0,65 | 391,105 | 1 | 601,7 | 1 | 601,7 |
| 23.99910002 | 0 | 1 | 0 | 1 | 0 | 1 | 0 |
| 24.99910032 | 2530,19 | 1 | 2530,19 | 1 | 2530,19 | 1 | 2530,19 |
| 25.99910407 | 0 | 1 | 0 | 1 | 0 | 1 | 0 |
| 26.99910483 | 1897,03 | 1 | 1897,03 | 0,78 | 1479,6834 | 1 | 1897,03 |
| 27.99910554 | 8095,41 | 1 | 8095,41 | 1 | 8095,41 | 1 | 8095,41 |
| 28.99910883 | 231,38 | 0,81 | 187,4178 | 1 | 231,38 | 1,71 | 395,6598 |
| 29.99911002 | 1913,47 | 1 | 1913,47 | 1 | 1913,47 | 1 | 1913,47 |
| 30.99911193 | 1263,31 | 1,26 | 1591,7706 | 1 | 1263,31 | 1 | 1263,31 |
| 31.99911415 | 316,09 | 1 | 316,09 | 1 | 316,09 | 1 | 316,09 |
| 32.99911559 | 1267,45 | 1 | 1267,45 | 1 | 1267,45 | 1,33 | 1685,7085 |
| 33.99911777 | 146,72 | 1 | 146,72 | 1 | 146,72 | 1 | 146,72 |
| 34.99912101 | 656,51 | 1 | 656,51 | 1 | 656,51 | 1 | 656,51 |
| 35.99912211 | 588,78 | 1,78 | 1048,0284 | 1 | 588,78 | 1 | 588,78 |
| 36.99912366 | 454,24 | 1 | 454,24 | 1,5 | 681,36 | 1 | 454,24 |
| 37.99912411 | 343,73 | 1 | 343,73 | 1 | 343,73 | 1 | 343,73 |
| 38.99912661 | 4149,61 | 1 | 4149,61 | 1 | 4149,61 | 1 | 4149,61 |
| 39.99913200 | 375,62 | 1 | 375,62 | 1,21 | 454,5002 | 1 | 375,62 |
| 40.99913322 | 0 | 1 | 0 | 1 | 0 | 1,86 | 0 |
| 41.99913825 | 1135,47 | 0,68 | 772,1196 | 0,65 | 738,0555 | 1 | 1135,47 |
| 42.99914211 | 635,96 | 1 | 635,96 | 1 | 635,96 | 1 | 635,96 |
| 43.99914445 | 163,97 | 1 | 163,97 | 1 | 163,97 | 0,64 | 104,9408 |
| 44.99914876 | 641,34 | 1 | 641,34 | 1,5 | 962,01 | 1 | 641,34 |
| 45.99914887 | 0 | 1 | 0 | 1 | 0 | 1 | 0 |
| 46.99914893 | 0 | 0,67 | 0 | 1 | 0 | 1 | 0 |
| 47.99914935 | 58,85 | 1 | 58,85 | 0,72 | 42,372 | 1,55 | 91,2175 |
| 48.99915015 | 0 | 1,83 | 0 | 1 | 0 | 1 | 0 |
| 49.99915755 | 0 | 0,67 | 0 | 1 | 0 | 1 | 0 |
| 50.99915778 | 105,04 | 1 | 105,04 | 1 | 105,04 | 1 | 105,04 |
| 51.99915883 | 147,94 | 1 | 147,94 | 1 | 147,94 | 1 | 147,94 |
| 52.99916170 | 2954,99 | 1,27 | 3752,8373 | 1 | 2954,99 | 1 | 2954,99 |
| 53.99916177 | 10106,78 | 1 | 10106,78 | 1,88 | 19000,7464 | 1 | 10106,78 |
| 54.99916611 | 0 | 1 | 0 | 1 | 0 | 0,79 | 0 |
| 55.99916615 | 0 | 1 | 0 | 1 | 0 | 0,83 | 0 |
| 56.99916966 | 1033,15 | 1 | 1033,15 | 1 | 1033,15 | 1 | 1033,15 |
| 57.99917047 | 6484,27 | 0,64 | 4149,9328 | 0,82 | 5317,1014 | 1 | 6484,27 |
| 58.99917221 | 49,54 | 0,74 | 36,6596 | 1 | 49,54 | 1 | 49,54 |
| 59.99917280 | 943,09 | 1 | 943,09 | 1 | 943,09 | 1 | 943,09 |
| 60.99917288 | 1468,46 | 1 | 1468,46 | 1 | 1468,46 | 1 | 1468,46 |
| 61.99917419 | 416,27 | 1 | 416,27 | 1 | 416,27 | 1 | 416,27 |
| 62.99917505 | 261,69 | 1 | 261,69 | 1 | 261,69 | 1 | 261,69 |
| 63.99917596 | 560,74 | 1 | 560,74 | 1 | 560,74 | 1 | 560,74 |
| 64.99917626 | 656,75 | 1 | 656,75 | 1 | 656,75 | 1 | 656,75 |
| 65.99917690 | 2383,49 | 1 | 2383,49 | 1 | 2383,49 | 0,63 | 1501,5987 |
| 66.99917825 | 257,55 | 1 | 257,55 | 1 | 257,55 | 2,01 | 517,6755 |
| 67.99917890 | 825,93 | 1 | 825,93 | 1 | 825,93 | 1 | 825,93 |
| 68.99917947 | 567,78 | 1 | 567,78 | 1 | 567,78 | 1 | 567,78 |
| 69.99918831 | 201,86 | 1 | 201,86 | 1,27 | 256,3622 | 1 | 201,86 |
| 70.99918906 | 295,19 | 1 | 295,19 | 1 | 295,19 | 1 | 295,19 |
| 71.99918909 | 127,29 | 1 | 127,29 | 0,62 | 78,9198 | 1 | 127,29 |

A new score for the CD71 classifier, based on the predicted levels after therapy with drugs A, B, and C can be calculated. A score of 1.611 is calculated for the prediction for drug A, 1.447 for drug B, and 1.845 for drug C. The results predict no impact of drug A, a negative impact, an increase of risk of death to 4.44% from drug B, and a decrease of the risk of death to 2.65% as a result of treatment with drug C. Based on this approach, treatment with drug C will be suggested to reduce the risk of death within 5 years.

The methods applied are based on standard protocols as identified, which are described for example in
1. Mavrogeorgis, E., Mischak, H., Latosinska, A., Siwy, J., Jankowski, V., and Jankowski, J. (2021) Molecules. 26**,**
2. Mischak, H., Vlahou, A., and loannidis, J. P. (2013) Clin. Biochem. 46, 432-443

## Claims

1. A method for determining the therapy regimen and/or the clinical outcome and/or the treatment success of a treatment of an individual with an active agent, comprising the steps of
a) Providing a body fluid sample from the individual;
b) Determining the peptidome/proteome in said sample from said individual;
c) Calculating of a reference value based on processing information of a predetermined group of peptide/protein markers of the peptidome/proteome determined in step b);
d) Determining a score for the predetermined group of peptide/protein markers of step c), whereby the score is calculated by processing information for each marker of the group obtained under the impact of the active agent, whereby the information is present in a database;
e) Processing the value obtained in step c) with the score obtained in step d);
f) Determining a treatment regimen and/or predicting the clinical outcome and/or predicting the treatment success with the active agent based on the processing in step e), whereby if the score is below the reference value, the treatment success is positive and/or the therapeutic regimen shall be applied and/or the clinical outcome is favorable.

2. Method for the prediction of a very relevant health event for an individual and the determination of possible preventive treatment of said relevant health event in the individual, comprising firstly, the steps for the probability of a very relevant health event by
a) Providing a body fluid sample from the individual;
b) Determining the peptidome/proteome in said sample;
c) Comparing the information on the peptidome/proteome obtained in step b) with information on the peptidome/proteome present in a database;
d) Determining the probability of a very relevant health event for an individual based on the comparison in step c); and, secondly,
e) Calculating of a reference value based on processing information of a predetermined group of peptide/protein markers determined in step b);
f) Determining a score for the predetermined group of peptide/protein markers used in step c), whereby the score is calculated by processing information for each marker of the group obtained under the impact of the active agent, whereby the information is present in a database;
g) Processing the value obtained in step e) with the score obtained in step f);
h) Determining a treatment regimen and/or predicting the clinical outcome and/or predicting the treatment success with the active agent based on the processing in step g), whereby if the score is below the reference value, the treatment success is positive and/or the therapeutic regimen shall be applied and/or the clinical outcome is favorable.

3. The method according to claim 2, wherein the step of comparison according to step c) includes the application of a classifier, comprising at least three of predetermined peptide/protein markers.

4. The method according to any one of the preceding claims, wherein at least one of steps c) to g) according to claim 2 or at least one of steps c) to e) according to claim 1 are conducted with an artificial intelligence routine including neural network, machine learning algorithm, in particular, support vector machine.

5. The method according to claim 2, wherein the individual is an apparently healthy individual or the individual is afflicted with a known disease.

6. The method according to any one of the preceding claims, wherein the comparison or calculation comprises an evaluation of the determined presence or absence or amplitude of peptide/protein markers.

7. The method according to any one of the preceding claims, wherein said sample of the individual is a urine sample, in particular, a midstream urine sample.

8. The method according to any one of the preceding claims, wherein capillary electrophoresis, HPLC, gas-phase ion spectrometry and/or mass spectrometry is used for determining the peptide/protein, in particular, the peptide/protein markers present in the sample.

9. The method according to any one of the preceding claims, wherein the peptide/protein determined in the peptide/protein sample includes collagen derived peptides/proteins, in particular, collagen α-1 derived peptides/proteins.

10. The method according to any one of the claims 2 to 9, wherein the very relevant health event is selected from major cardiovascular events, kidney failure, tumor recurrence or death as a result of a disease.

11. The method according to any one of the preceding claims, wherein the method predicts a treatment success with a predetermined active agent *in silico* for stratification of the therapeutic regimen.

12. The method according to any one of the claims 2 to 9 for determining the therapy end with an active agent, wherein according to step f) when the score is above the reference value (classifier), the administration of the active agent does not have any additional therapeutic effects.

13. The method according to any one of the preceding claims, wherein the information of the peptidome/proteome, in particular, the predetermined group of peptide/protein markers include values for the molecular masses and migration times, in particular, obtained by CE-MS and/or the information includes an adjusted p-value for the association with the predefined outcome.

14. The use of a test kit or kit of parts for the prediction of a very relevant health event for an individual; for determining the therapeutic regimen; for predicting the clinical outcome; and/or for predicting the treatment success of a treatment for an individual, respectively, said kit comprising means for determining the peptidome/proteome present in a body fluid sample and, optionally, means for reprocessing of the body fluid sample before determining the peptidome/proteome and instructions on how to use said test kit or kit of parts for a method according to any one of claims 1 to 13.

15. A computer implemented method of prediction of a very relevant health event for an individual; for determining the therapeutic regimen; for predicting the clinical outcome; and/or for predicting the treatment success of a treatment for an individual with an active agent, respectively, comprising the steps of
a) Obtaining information of the peptidome/proteome in a body fluid sample of the individual;
b) Computing the information of step a) by
i. Comparing the information on the peptidome/proteome obtained in step a) with information on the peptidome/proteome obtained from a database; or
ii. by calculating of a reference value based on the information of a predetermined group of peptide/protein markers and computing a score for the predetermined group of peptide/protein markers, whereby the score is calculated from information for each marker of the group under the influence of the active agent, whereby the information is obtained from a database, comparing the computed reference value with the score,
c) Determining the probability of a very relevant health event of an individual; or determining a treatment regimen and/or predicting the clinical outcome and/or predicting the treatment success with the active agent, respectively;
d) Optionally further comprising the step of showing the data of prediction of a very relevant health event for an individual, treatment regimen, predicting the clinical outcome and/or predicting the treatment success with the active agent, respectively, on an output unit.

16. A computer readable medium or computer program product having computer executable instructions for performing the steps as identified in any one of the claims 1 to 13.
